# EUROPEAN PATENT APPLICATION

(11) **EP 4 657 279 A1**
(43) Date of publication of application: **03.12.2025**
(21) Application number: 25178496.3
(22) Date of filing: 23.05.2025
(51) Int. Cl.: G06F 16/332, G06F 16/3329, G06F 16/334, G16H 50/20, G16H 50/70

(54) **MEDICAL INFORMATION PROCESSING APPARATUS, MEDICAL INFORMATION PROCESSING METHOD, AND MEDICAL INFORMATION PROCESSING PROGRAM**

(30) Priority: 27.05.2024 JP 2024085595
(71) Applicant: Canon Medical Systems Corporation, Tochigi 324-0036 (JP)
(72) Inventor: NEGISHI, Takuo, Otawara-shi, 324-0036 (JP); ARITA, Kosuke, Otawara-shi, 324-0036 (JP); KIMURA, Asateru, Otawara-shi, 324-0036 (JP); SUGIYAMA, Atsuko, Otawara-shi, 324-0036 (JP)
(74) Representative: Marks & Clerk LLP

(57) **Abstract**

A medical information processing apparatus (30) according to one embodiment includes a generator unit (34a), an obtaining unit (34b), and an output unit (34c). The generator unit (34a) generates a prompt to be input to a first generative model (11), in accordance with a user's input. The obtaining unit (34b) obtains an answer including medical information from the first generative model (11), in response to an input of the prompt to the first generative model (11). The output unit (34c) outputs reliability of the answer based on the prompt and the answer.

## Description

### FIELD

Embodiments described herein relate generally to a medical information processing apparatus, a medical information processing method, and a medical information processing program.

### BACKGROUND

Conventionally, generative models (hereinafter, referred to as large language models (LLMs) have been applied in the field of medicine, such as using an LLM for automated generation of questions to ask physicians. LLMs may however output answers that provide untrue information or are completely incoherent with or irrelevant to the context. Such a phenomenon is called hallucinations.

As an example, there is a technique for causing a computer to obtain technical information including a technical idea and patent information to be compared with the technical information and input an instruction designating the technical information and the patent information into a generative model, to output an evaluation result indicating relevance between the technical information and the patent information based on the result generated by the generative model. This technique presents not the reliability of LLM answers but room for improvement in reliability.

### SUMMARY OF THE INVENTION

A medical information processing apparatus is provided, which includes a generator unit configured to generate a prompt to be input to a first generative model, in accordance with a user's input; an obtaining unit configured to obtain an answer including medical information from the first generative model, in response to an input of the prompt to the first generative model; and an output unit configured to output reliability of the answer based on the prompt and the answer.

The output unit may extract a first named entity from the prompt; extract a second named entity from the answer; search a medical information database using the first named entity and the second named entity as a search condition, to identify document data based on how many times the first named entity and the second named entity appear; calculate a similarity of the document data relative to the prompt and the answer, based on a first vector and a second vector, the first vector representing a vectorization of the document data, the second vector representing a vectorization of the prompt and the answer; and determine the reliability based on the similarity.

The output unit may identify document data in which the first named entity and the second named entity appear a largest number of times.

The output unit may extract a first named entity from the prompt, extract a second named entity from the answer, search a medical information database using the first named entity and the second named entity as a search condition, to calculate a hit count under the search condition, and determine the reliability based on the hit count.

The output unit may extract a first named entity from the prompt, extract a second named entity from the answer, calculate a number of mutually corresponding named entities in the first named entity and the second named entity, and determine the reliability based on the number of mutually corresponding named entities.

The generator unit may generate an inquiry prompt for inquiring about reliability of the answer, based on the prompt and the answer. The obtaining unit may obtain an output from the first generative model or a second generative model different from the first generative model, in response to an input of the inquiry prompt to the first generative model or the second generative model. The output unit may output the reliability in accordance with the obtained output from the first generative model or the second generative model.

The generator unit may generate an inquiry prompt for inquiring about reliability of the answer. The obtaining unit may obtain an output from the first generative model or a second generative model different from the first generative model, in response to an input of the inquiry prompt, the prompt, and the answer to the first generative model or the second generative model. The output unit may output the reliability in accordance with the obtained output from the first generative model or the second generative model.

The medical information processing apparatus may further include a display unit that displays the reliability and a basis for the reliability.

The medical information processing apparatus may further include a display unit that displays the prompt and the answer with the reliability.

A medical information processing method is provided, which includes generating a prompt to be input to a generative model, in accordance with a user's input; inputting the prompt to the generative model; obtaining an answer including medical information from the generative model; and outputting reliability of the answer based on the prompt and the answer.

A medical information processing program is provided, which causes a computer to perform generating a prompt to be input to a generative model, in accordance with a user's input; inputting the prompt to the generative model; obtaining an answer including medical information from the generative model; and outputting reliability of the answer based on the prompt and the answer.

### BRIEF DESCRIPTION OF THE DRAWINGS

FIG. 1 is a schematic block diagram illustrating an exemplary configuration of a medical information processing system including a medical information processing apparatus according to an embodiment;
FIG. 2 illustrates an example of a prompt, an LLM's answer for the prompt, reliability, and a basis for the reliability according to an embodiment;
FIG. 3 is a flowchart illustrating steps of a reliability determining process according to an embodiment;
FIG. 4 illustrates an example of answer reliability as displayed by a fact-check function for comparison with an embodiment;
FIG. 5 illustrates an example of a problem in answer reliability as displayed by the fact-check function for comparison with an embodiment;
FIG. 6 is a flowchart illustrating steps of a reliability determining process according to a first modification of some embodiments;
FIG. 7 is a flowchart illustrating steps of a reliability determining process according to a second modification of some embodiments;
FIG. 8 is a flowchart illustrating steps of a reliability determining process according a third modification of some embodiments;
FIG. 9 is a schematic block diagram illustrating an exemplary configuration of an X-ray CT apparatus according to a second application of some embodiments; and
FIG. 10 illustrates an example of a prompt, an LLM's answer for the prompt, reliability, and a basis for the reliability according to the second application of some embodiments.

### DETAILED DESCRIPTION

One of the problems to be solved by some embodiments disclosed in this specification and the accompanying drawings is to improve the reliability of answers of a generative model applied to the field of medicine, in terms of accuracy. However, the problems to be solved by some embodiments disclosed in this specification and the accompanying drawings are not limited to such a problem. Problems to be dealt with by effects of the respective features described in the following embodiments can be considered as other problems.

According to an embodiment, a medical information processing apparatus includes a generator unit, an obtaining unit, and an output unit. The generator unit generates a prompt to be input to a first generative model, in accordance with a user's input. The obtaining unit obtains an answer including medical information about the user from the first generative model, in response to an input of the prompt to the first generative model. The output unit (34c) outputs reliability of the answer based on the prompt and the answer.

Hereinafter, exemplary embodiments of an X-ray CT apparatus, a medical information processing apparatus, a medical information processing system, an information processing apparatus, a medical information processing method, an information processing method, and a medical information processing program will be described in detail with reference to the accompanying drawings. Throughout this disclosure, parts or elements given the same reference numerals are considered to perform similar operations, and redundant descriptions thereof will be omitted when appropriate.

### Embodiment

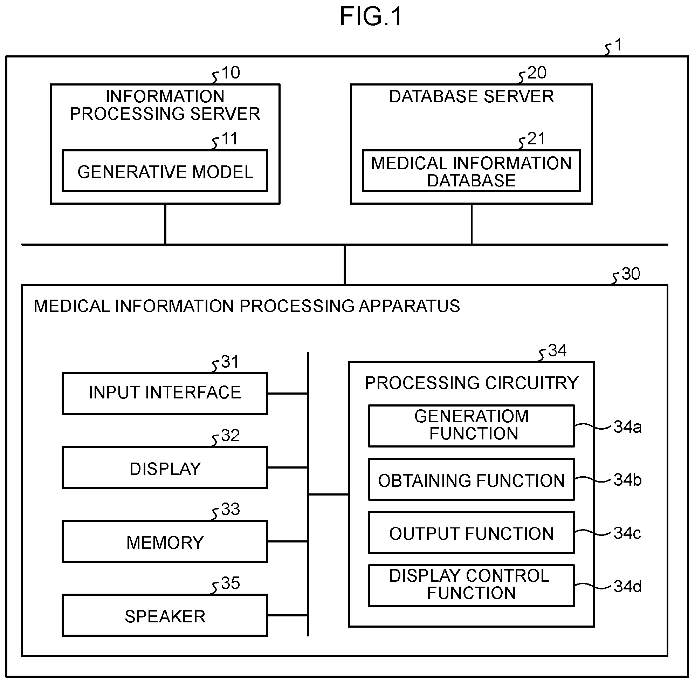

FIG. 1 is a schematic block diagram illustrating an exemplary configuration of a medical information processing system 1 including a medical information processing apparatus 30 according to an embodiment. As illustrated in FIG. 1, the medical information processing system 1 of an embodiment includes an information processing server 10, a database server 20, and a medical information processing apparatus 30. The information processing server 10 and the database server 20 are connected to the medical information processing apparatus 30 via a network such as the Internet and/or intranet. Alternatively, the medical information processing apparatus 30 may be connected to other servers and/or other databases, in addition to the information processing server 10 and the database server 20.

The information processing server 10 includes memory storing a generative model 11, and a processor that executes the generative model 11. The generative model 11 represents, for example, a large language model (LLM) or a multimodal model (MMM). Herein, the generative model 11 is exemplified by an LLM but the LLM 11 may be replaced with another model as an MMM. The LLM 11 is an artificial intelligence (AI) model which has been pre-trained using a large-scale corpus in the field of natural language processing. For example, the LLM 11 is trained to receive a sentence (prompt) signifying a question or an instruction as an input and generate and output a reply in line with the meaning of the input prompt.

The processor in the information processing server 10 retrieves the LLM 11 from the memory and inputs, into the LLM 11, the prompt output from the medical information processing apparatus 30 to cause the LLM 11 to generate a reply. The processor transmits the generated reply (output of the LLM 11) to the medical information processing apparatus 30. The generated reply, **i.e.,** the LLM 11's answer for the prompt, contains medical information as to a user. The LLM 11 may be incorporated in the medical information processing apparatus 30.

The database server 20 includes memory storing the medical information database 21. The medical information database 21 contains various kinds of medical information such as medical papers and columns and reports published in medical journals. For instance, the medical information database 21 may be stored in a hospital information system (HIS). In such a case the medical information database 21 contains various kinds of medical information stored in the HIS. Also, the medical information database 21 may further contain information as to examinations, diagnoses, medical treatments, and procedures, in addition to various kinds of medical information.

The medical information processing apparatus 30 transmits prompts to the information processing server 10 via the network. The medical information processing apparatus 30 obtains replies output from the LLM 11 from the information processing server 10 via the network. The medical information processing apparatus 30 also accesses the database server 20 via the network to search the medical information database 21. Alternatively, the processor of the database server 20 may search the medical information database 21 in accordance with a search condition output from the medical information processing apparatus 30. The medical information processing apparatus 30 obtains search results from the database server 20.

The medical information processing apparatus 30 can be implemented by, for example, any of various kinds of terminals that the user uses. Examples of various kinds of terminals include a network-connectable smart phone, various personal computers, and a tablet terminal. In addition, the medical information processing apparatus 30 may be implemented by a terminal device disposed in a hospital. As illustrated in FIG. 1, the medical information processing apparatus 30 includes an input interface 31, a display 32, a memory 33, processing circuitry 34, and a speaker 35.

The input interface 31 can be, for example, implemented by a trackball, a switch, a button, a mouse, and a keyboard, a touchpad that allows input by touch on the operation surface, a touch screen as an integration of a display screen and a touchpad, non-contact input circuitry including an optical sensor, and audio input circuitry (e.g., a microphone), for allowing the user to give various kinds of instructions and perform various kinds of setting. The input interface 31 receives an input from the user and converts the input to an electric signal for output to the processing circuitry 34. For example, the input interface 31 receives a question for the LLM 11 with audio or characters according to a user instruction.

The input interface 31 is not limited to the one including physical operational component or components as a mouse and a keyboard. Other examples of the input interface 31 include electrical-signal processing circuitry that receives an electrical signal corresponding to an input from an external input device separated from the medical information processing apparatus 30 to output the electrical signal to the processing circuitry 34. The input interface 31 is an example of an input unit and may be referred to as an operational unit.

The display 32 displays various kinds of information under the control of a display control function 34d. For example, the display 32 displays a graphical user interface (GUI) that allows the user to input instructions, prompts to the LLM 11, answers from the LLM 11, the reliability of the answers, and bases for the answers. For example, the display 32 can be a liquid crystal display (LCD) or a cathode ray tube (CRT) display. The display 32 is an example of a display unit.

The memory 33 can be, for example, implemented by a semiconductor memory device as random access memory (RAM) or flash memory, a hard disk, or an optical disk. The memory 33 stores, for example, different kinds of data generated by various kinds of processing of the processing circuitry 34 as described later. The memory 33 further stores replies (answers) output from the LLM 11. The memory 33 stores results of searching the medical information database 21. Also, the memory 33 stores various kinds of computer programs that cause the respective circuits included in the medical information processing apparatus 30 to implement the respective functions. The memory 33 is an example of a storage unit.

The processing circuitry 34 controls the operation of the medical information processing apparatus 30 as a whole by performing a generation function 34a, an obtaining function 34b, an output function 34c, and the display control function 34d. The processing circuitry 34 implementing the generation function 34a corresponds to a generator unit. The processing circuitry 34 implementing the obtaining function 34b corresponds to an obtaining unit. The processing circuitry 34 implementing the output function 34c corresponds to an output unit. The processing circuitry 34 implementing the display control function 34d corresponds to a display control unit.

The processing circuitry 34 retrieves and executes the program corresponding to the generation function 34a from the memory 33. This allows the generation function 34a to generate a prompt to be input to the LLM 11, in response to a user input given via the input interface 31. The prompt generation responsive to an input from the input interface 31 can be implemented by any known method, therefore, a description thereof is omitted. The generation function 34a stores the generated prompt in the memory 33.

The processing circuitry 34 retrieves and executes the program corresponding to the obtaining function 34b from the memory 33. This allows the obtaining function 34b to transmit the generated prompt to the information processing server 10. The LLM 11 generates an answer upon an input of the prompt and the obtaining function 34b obtains the answer from the LLM 11.

Specifically, the processor of the information processing server 10 inputs the generated prompt into the LLM 11 to generate an answer as an output of the LLM 11. The obtaining function 34b obtains the answer output from the LLM 11 from the information processing server 10. The answer output from the LLM 11 is responsive to the prompt input to the LLM 11.

The processing circuitry 34 retrieves and executes the program corresponding to the output function 34c from the memory 33. This allows the output function 34c to determine reliability of the answer based on the prompt and the answer. Specifically, the output function 34c extracts a named entity from the prompt through natural language processing (hereinafter, the named entity extracted from the prompt is referred to as a first named entity). As an example, the prompt may be a sentence such as "I'm a 30-year old male and found a lump on my neck. Is this some kind of disease?". In this case the first named entity can be represented by, for example, "gender: male, age: 30, site: neck, lesion: lump".

The output function 34c also extracts a named entity from the answer of the LLM 11 through natural language processing (hereinafter, the named entity extracted from the answer of the LLM 11 is referred to as a second named entity). For example, the answer may be a sentence such as "A lump on the neck is usually benign so please observe the progress". In this case the second named entity can be represented by, for example, "site: neck, lesion: lump".

Named entities can be extracted by known natural language processing, therefore, a description thereof is omitted. The extracted named entities may be classified in units of items, as described above. Also, data on the extracted named entities may be structuralized, as described above. In addition, the processing circuitry 34 may extract the first named entity from the prompt and extract the second named entity from the answer as a separate extraction function.

The processing circuitry 34 uses the output function 34c to search the medical information database 21 using the first named entity and the second named entity as a search condition. Through the search, the output function 34c identifies document data in the medical information database 21 based on how many times the first named entity and the second named entity appear therein. For example, the output function 34c identifies document data in which the first named entity and the second named entity appear a largest number of times. The output function 34c then obtains the identified document data from the medical information database 21. The output function 34c stores the obtained document data in the memory 33 in association with the first named entity and the second named entity.

The processing circuitry 34 then uses the output function 34c to vectorize the identified document data. Thus, the output function 34c subjects the identified document data to vectorization in natural language processing, thereby generating a vector for the document data (hereinafter, a first vector). The first vector indicates a feature amount (text feature amount) of the identified document data.

The output function 34c further vectorizes the prompt and the answer. Namely, the output function 34c subjects the prompt and answer to vectorization in natural language processing, thereby generating a vector for the prompt and answer (hereinafter, a second vector). The second vector indicates a text feature amount of a combination of the prompt and the answer.

The vectorization in natural language processing can be implemented by known natural language processing or a known trained model, therefore, a description thereof is omitted. Further, the processing circuitry 34 may perform the vectorization of document data into the first vector and the vectorization of a prompt and an answer into the second vector as a separate vectorization function.

The processing circuitry 34 uses the output function 34c to calculate a similarity of the document data relative to the prompt and answer, based on the first vector and the second vector. Specifically, the output function 34c calculates a distance between the first vector and the second vector. Such a distance represents, for example, a Mahalanobis' distance in a vector space. A shorter Mahalanobis' distance means that the first vector and the second vector are closer to each other, so that the output function 34c calculates a larger similarity as the Mahalanobis' distance shortens. The output function 34c stores the resultant similarity in the memory 33.

Alternatively, the output function 34c may calculate a reciprocal of the Mahalanobis' distance as a similarity, for example. The similarity calculation is not limited to the Mahalanobis' distance calculation as above, and any method of calculating vector similarity may be applicable. In addition, the processing circuitry 34 may perform the similarity calculation for the first vector and the second vector as a separate similarity calculation function.

The processing circuitry 34 uses the output function 34c to determine reliability of the answer based on the similarity. For example, when the reliability is classified into three levels, low, medium, and high, a first threshold representing a boundary between low reliability and medium reliability and a second threshold representing a boundary between medium reliability and high reliability are preset and stored in the memory 33. The first threshold and the second threshold can be freely set and changed when appropriate. The number of classifications can be set to any number other than three. In this case the number of thresholds equal to (the number of classifications - 1) is set in advance. In the following the number of classifications is defined as three for the sake of specificity. In the present embodiment, the thresholds are used to determine how much two kinds of document data, **i.e.,** the identified document data and the document data based on the prompt and the answer, are similar to each other in terms of contents (hereinafter, referred to as a similarity determination threshold).

The processing circuitry 34 uses the output function 34c to compare the resultant similarity and a first similarity determination threshold. When the similarity is less than the first similarity determination threshold, the output function 34c determines the answer as having low reliability. When the similarity matches or exceeds the first similarity determination threshold, the output function compares the similarity and a second similarity determination threshold. When the similarity is less than the second similarity determination threshold, that is, the similarity matches or exceeds the first similarity determination threshold and is less than the second similarity determination threshold, the output function 34c determines the answer as having medium reliability. When the similarity matches or exceeds the second similarity determination threshold, the output function 34c determines the answer as having high reliability. The output function 34c then stores the resultant reliability and the answer in the memory 33 in association with each other.

The processing circuitry 34 retrieves and executes the program corresponding to the display control function 34d from the memory 33. For example, the display control function 34d displays the answer, the reliability, and a basis for the reliability together on the display 32. That is, the display 32 displays the answer and the reliability together with the basis for the reliability. At low reliability, the display control function 34d may further display a predetermined warning on the display 32.

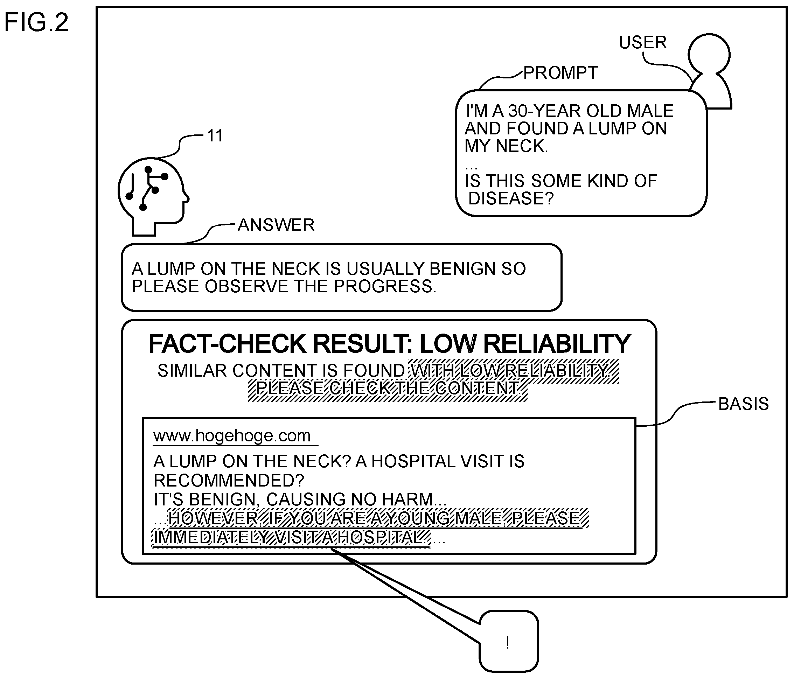

FIG. 2 illustrates an example of a prompt, an LLM 11's answer for the prompt, reliability, and a basis for the reliability. As illustrated in FIG. 2, the LLM 11's answer for the prompt contains medical information as to the user (e.g., benign, observe the progress in FIG. 2). As illustrated in FIG. 2, the basis for the reliability to be displayed may be, for example, a site name in the medical information database 21 and wording representing a basis. When the reliability of the answer is low, as illustrated in FIG. 2, a predetermined form of warning (exclamation mark) is added to the basis on display. This allows the user to easily understand the basis for the low reliability of the answer.

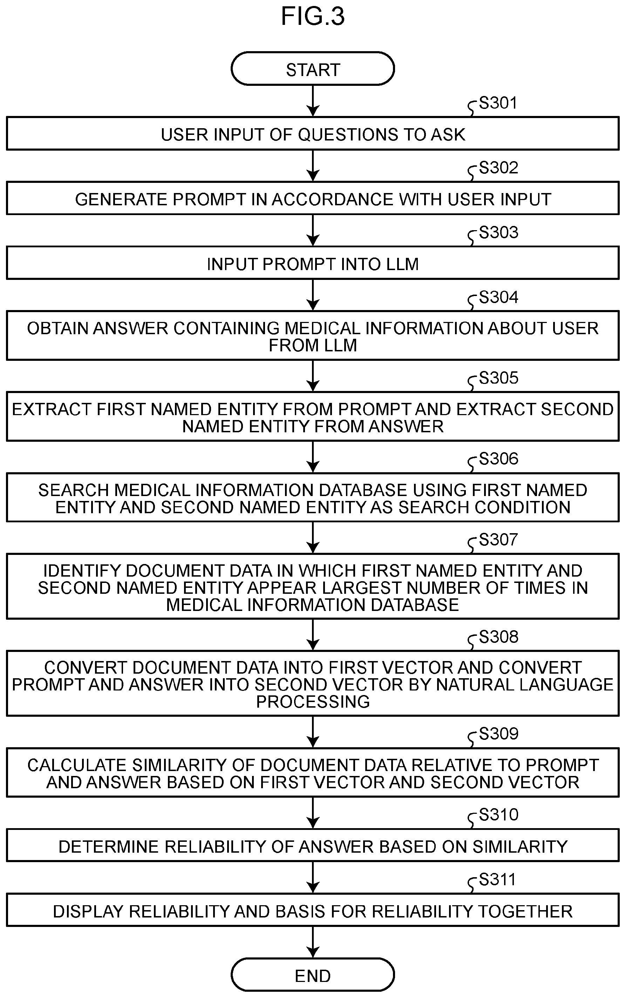

The overall configuration and structure of the medical information processing system 1 according to some embodiments have been explained as above. The following will describe a process of determining the reliability of an answer output from the LLM 11 (hereinafter, a reliability determining process). FIG. 3 is a flowchart illustrating steps of a reliability determining process by way of example. Reliability Determining Process

### Step S301

The user inputs some questions to ask the LLM 11 as a user instruction via the input interface 31. The questions are medical questions about the user.

### Step S302

The processing circuitry 34 uses the generation function 34a to generate a prompt to be input to the LLM 11 in accordance with the user input via the input interface 31. The generation function 34a stores the prompt in the memory 33.

### Step S303

The processing circuitry 34 uses the obtaining function 34b to transmit the prompt to the information processing server 10. Then, the information processing server 10 inputs the prompt to the LLM 11.

### Step S304

The information processing server 10 generates an answer for the input prompt as the output of the LLM 11. The processing circuitry 34 uses the obtaining function 34b to obtain, from the information processing server 10, the answer for the prompt output from the LLM 11. The obtaining function 34b stores the answer in the memory 33 in association with the prompt.

### Step S305

The processing circuitry 34 uses the output function 34c to extract a first named entity from the prompt and extract a second named entity from the answer. The output function 34c stores the first named entity in the memory 33 in association with the prompt. The output function 34c also stores the second named entity in the memory 33 in association with the answer of the LLM 11.

### Step S306

The processing circuitry 34 uses the output function 34c to search the medical information database 21 using the first named entity and the second named entity as a search condition. Alternatively, a search function included in the processing circuitry 34 may perform the search. The extent of the search is not limited to the medical information database 21 and the search may be conducted on the Internet.

### Step S307

The processing circuitry 34 uses the output function 34c to identify document data in the medical information database 21 based on how many times the first named entity and the second named entity appear therein. As an example, the output function 34c identifies document data in which the first named entity and the second named entity appear a largest number of times. The output function 34c obtains the identified document data from the medical information database 21. Alternatively, the obtaining function 34b may obtain the identified document data.

### Step S308

The processing circuitry 34 uses the output function 34c to convert the document data into a first vector by vectorization in natural language processing. For example, the output function 34c stores the first vector and the document data in the memory 33 in association with each other. The output function 34c also converts the prompt and the answer collectively into a second vector by vectorization in natural language processing. The output function 34c then stores the second vector and the prompt and answer in the memory 33 in association with each other.

### Step S309

The processing circuitry 34 uses the output function 34c to calculate a similarity of the document data relative to the prompt and answer, based on the first vector and the second vector. For example, the output function 34c calculates a distance between the first vector and the second vector in a feature vector space. The output function 34c calculates the similarity from the resultant distance.

The output function 34c calculates the similarity such that the closer the distance between the first vector and the second vector is, the larger the similarity obtained is. Also, the output function 34c calculates the similarity such that the farther the distance between the first vector and the second vector is, the smaller the similarity obtained is. Alternatively, the similarity may be calculated according to how far the first vector and the second vector are from each other (e.g., an inner product value), in place of according to the distance.

### Step S310

The processing circuitry 34 uses the output function 34c to determine reliability of the answer based on the similarity. Specifically, the output function 34c determines the reliability of the answer by comparing a preset similarity determination threshold and the similarity.

### Step S311

The processing circuitry 34 uses the display control function 34d to display the answer, the reliability, and a basis for the reliability together on the display 32. Thus, the display 32 displays the reliability of the answer together with the basis for the reliability, as illustrated in FIG. 2, for example. Further, the display 32 may display the prompt and answer together with the reliability. Also, the display control function 34d may display a predetermined warning on the display 32 as illustrated in FIG. 2. The predetermined warning may be, for example, in the form of bold face or highlighting. Alternatively, the display control function 34d may cause the speaker 35 to output a predetermined audio warning.

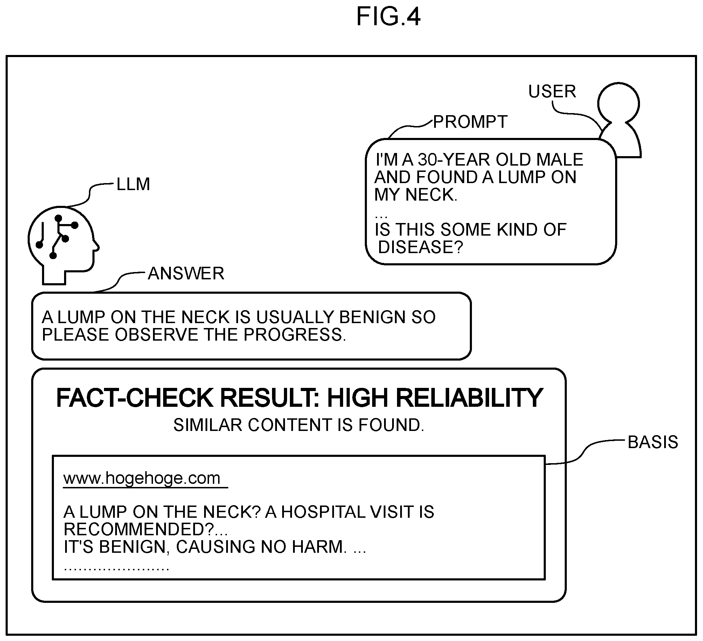

FIG. 4 illustrates an example of answer reliability displayed by a fact-check function for comparison with the present embodiment. As illustrated in FIG. 4, the fact-check function performs a fact check of the LLM's answer for the prompt based on the user input, to display a result of the fact check (fact-check result) and a basis for the fact check. FIG. 4 depicts a Website page on the Internet as an example of a basis for the fact check.

In this example, the comparative fact-check function presents the reliability of the comparative LLM's answer by providing information representing that "Information similar to the LLM's answer is found on the Internet", which is however insufficient. For example, based on the information similar to the LLM's answer found on the Internet, the fact-check function may end up with presenting, to the user, as a reliable answer, a user's unintended answer and/or an answer not reflecting the context and condition setting for the input to the LLM.

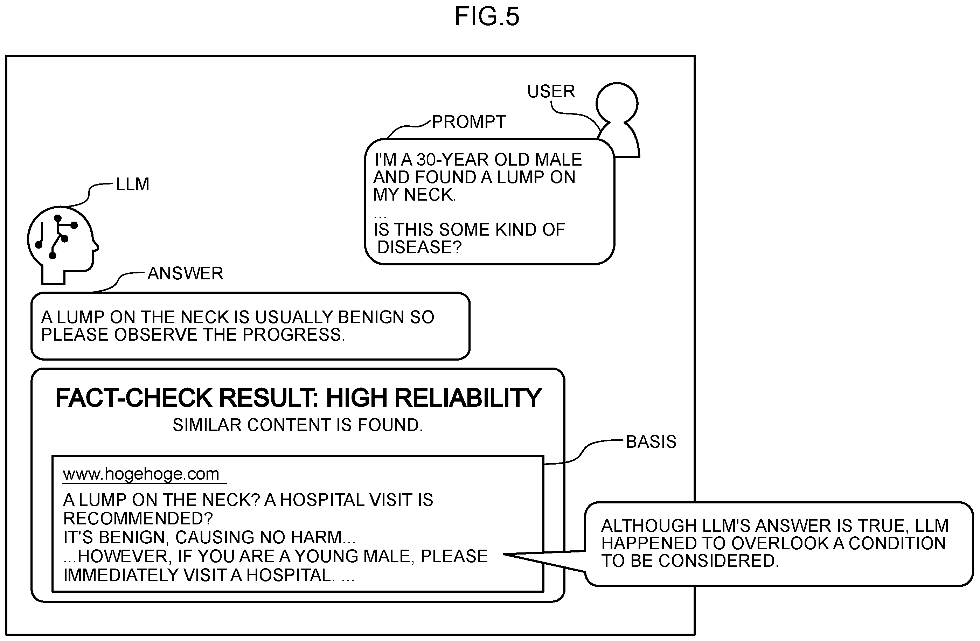

FIG. 5 illustrates an example of a problem in answer reliability as displayed by the fact-check function for comparison with the present embodiment. As illustrated in FIG. 5, when scrolling the website page in the fact-check result box, the sentence "..., but if you are a young male, please immediately visit a hospital..." appears. However, the LLM' s answer is such that "A lump on the neck is usually benign so please observe the progress" although the prompt describes "I'm a 30-year-old male." As such, the LLM's answer is true, however, the LLM happened to overlook the condition to be considered, "..., but if you are a young male, please immediately visit a hospital...", as illustrated in FIG. 5. That is, a hallucination is occurring here.

As illustrated in FIG. 5, there may be a situation that the reliability of the LLM's answer cannot be regarded as sufficient even if the fact-check function determines the LLM's answer to be appropriate. In particular, in the case of using the LLM in the medical field, LLM's answers may result in causing users to suffer detriments to their health.

In contrast, the medical information processing apparatus 30 according to an embodiment generates a prompt to be input to the LLM 11 in accordance with a user input; obtains an answer including medical information about the user from the LLM 11 in response to an input of the prompt to the LLM 11; and determines reliability of the answer based on the prompt and the answer. The medical information processing apparatus 30 according to an embodiment also displays the answer and the reliability together with a basis for the reliability.

Specifically, the medical information processing apparatus 30 of an embodiment extracts a first named entity from the prompt and extracts a second named entity from the answer to search the medical information database 21 using the first named entity and the second named entity as a search condition and identify document data in which the first named entity and the second named entity appear a largest number of times. The medical information processing apparatus 30 calculates a similarity of the document data relative to the prompt and the answer based on a first vector and a second vector, the first vector representing a vectorization of the document data, the second vector representing a vectorization of the prompt and the answer; and determines the reliability of the answer based on the similarity.

Owing to such features, unlike the comparative example, the medical information processing apparatus 30 of an embodiment can perform a fact check while taking the context and condition setting for the prompt based on a user input taken into account, by determining how much two kinds of document data, **i.e.,** the identified document data and the document data based on the prompt and the answer, are similar to each other in terms of contents. Thus, the medical information processing apparatus 30 of an embodiment performs a fact check of the identified document data using the prompt and the answer for the prompt, to be thereby able to determine whether the answer of the LLM is coherent with the context of the prompt. In this manner, the medical information processing apparatus 30 of an embodiment can improve the reliability of answers of the LLM 11 applied to the medical field in terms of accuracy, resulting in reducing detriments to the users' health.

### First Modification

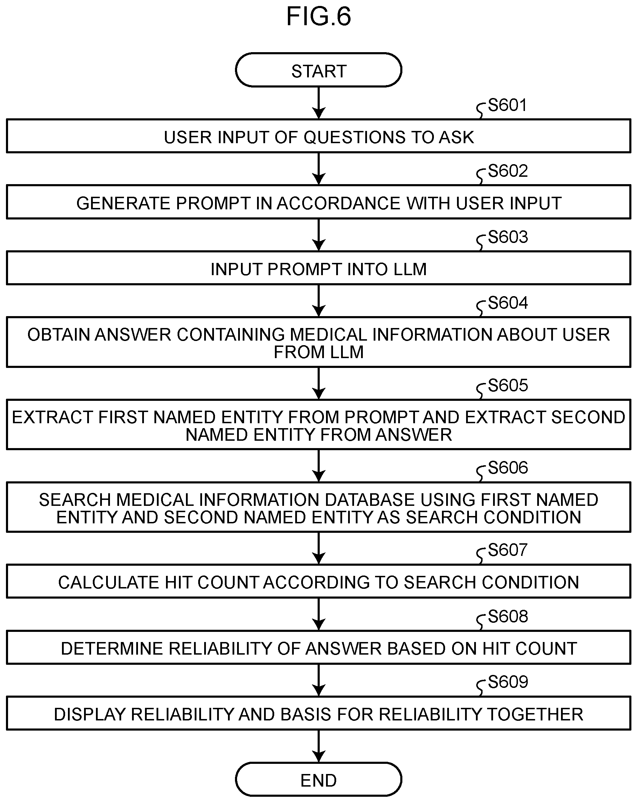

A first modification concerns searching the medical information database 21 and/or the Internet using the first named entity and the second named entity as a search condition to determine answer reliability depending on a hit count of literatures or documents or else in the search. The following will describe a reliability determining process in this modification, referring to FIG. **6****,** as an example.

FIG. 6 is a flowchart illustrating steps of a reliability determining process in the first modification by way of example. The processing in step S601 to step S606 and at step S609 in FIG. 6 is the same as the processing in step S301 to step S306 and at step S311 in FIG. 3 so that a description thereof is omitted.

### Reliability Determining Process

### Step S607

The processing circuitry 34 uses the output function 34c to calculate a hit count according to a search condition being the first named entity and the second named entity.
For example, the output function 34c counts (calculates) the number of hits of sets of document data including both the first named entity and the second named entity in the results of searching the medical information database 21 at Step S606. The output function 34c then stores the resultant hit count in the memory 33.

### Step S608

The processing circuitry 34 uses the output function 34c to determine answer reliability based on the hit count. Specifically, the output function 34c retrieves a threshold for classifying the answer reliability based on the hit count (hereinafter, a hit threshold) from the memory 33. The hit threshold is preset and stored in the memory 33. Hereinafter, the number of classifications based on the hit count are defined as three for the sake of specificity. Two kinds of hit threshold, **i.e.,** a first hit threshold and a second hit threshold greater than the first hit threshold, are thus retrieved from the memory 33.

The output function 34c compares the hit count and the first hit threshold. When the hit count is less than the first hit threshold, the output function 34c determines the answer as having low reliability. When the hit count matches or exceeds the first hit threshold, the output function 34c compares the hit count and the second hit threshold. When the hit count is less than the second hit threshold, **i.e.,** the hit count matches or exceeds the first hit threshold and is less than the second hit threshold, the output function 34c determines the answer as having medium reliability. When the hit count matches or exceeds the second hit threshold, the output function 34c determines the answer as having high reliability. The output function 34c stores the determined reliability and the answer in the memory 33 in association with each other.

As described above, the medical information processing apparatus 30 according to the first modification of some embodiments extracts a first named entity from the prompt and extracts a second named entity from the answer to search the medical information database 21 using the first named entity and the second named entity as a search condition to calculate a hit count according to the search condition and determine reliability of the answer based on the hit count. In this manner, the medical information processing apparatus 30 according to the first modification of some embodiments can determine answer reliability in accordance with the hit count in the results of searching the medical information database 21 under the search condition being the first named entity and the second named entity. Other effects of this modification are similar to or the same as those of some embodiments, therefore, a description thereof is omitted.

### Second Modification

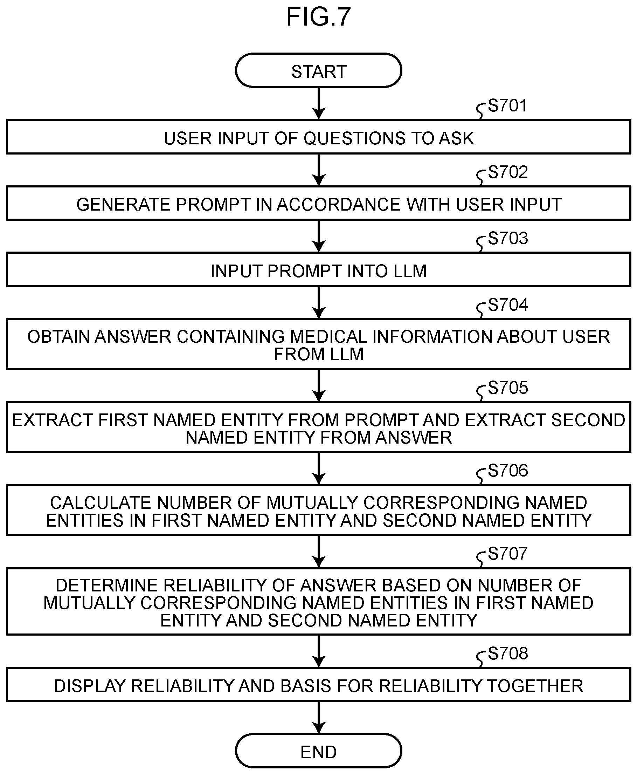

A second modification concerns determining answer reliability through comparison between the number of attributes included in the first named entity and the number of attributes included in the second named entity. The attributes refer to, for example, words such as adjectives and nouns and may be referred to as elements. The following will explain a reliability determining process according to a second modification, referring to FIG. **7****.**

FIG. 7 is a flowchart illustrating steps of a reliability determining process in the second modification by way of example. In the flowchart of FIG. **7****,** the processing in step S701 to step S705 and step S708 is the same as the processing in step S301 to step S305 and step S311 of FIG. **3****,** therefore, a description thereof is omitted.

### Reliability Determining Process

### Step S706

The processing circuitry 34 uses the output function 34c to calculate the number of mutually corresponding named entities in the first named entity and the second named entity. Specifically, the output function 34c calculates (counts) the number of attributes contained in the first named entity (hereinafter, a first attribute number). The first attribute number may also be referred to as a first element number. The output function 34c stores the first attribute number in the memory 33 in association with the prompt.

The processing circuitry 34 further uses the output function 34c to calculate (count) the number of attributes contained in the second named entity (hereinafter, a second attribute number). The second attribute number may also be referred to as a second element number. The output function 34c then stores the second attribute number in the memory 33 in association with the answer.

### Step S707

The processing circuitry 34 uses the output function 34c to determine reliability of the answer based on the number of mutually corresponding named entities in the first named entity and the second named entity. Specifically, the output function 34c compares the first attribute number and the second attribute number. The output function 34c determines reliability of the answer as high when the first attribute number and the second attribute number are close to each other. When the first attribute number and the second attribute number are far from each other, the output function 34c determines reliability of the answer as low. Thus, the output function 34c determines the answer reliability based on a difference between the first attribute number and the second attribute number (hereinafter, referred to as an attribute difference).

Specifically, the output function 34c retrieves from the memory 33 a threshold for classifying the answer reliability based on an attribute difference (hereinafter, an attribute threshold). The attribute threshold is preset and stored in the memory 33. In the following the number of classifications based on the attribute difference is defined as three for the sake of specificity. Two kinds of attribute threshold, **i.e.,** a first attribute threshold and a second attribute threshold greater than the first attribute threshold, are retrieved from the memory 33.

The output function 34c compares the attribute difference and the first attribute threshold. When the attribute difference is less than the first attribute threshold, the output function 34c determines the answer as having high reliability. When the attribute difference matches or exceeds the first attribute threshold, the output function 34c compares the attribute difference and the second attribute threshold. When the attribute difference is less than the second attribute threshold, that is, the attribute difference matches or exceeds the first attribute threshold and is less than the second attribute threshold, the output function 34c determines the answer as having medium reliability. When the attribute difference matches or exceeds the second attribute threshold, the output function 34c determines the answer as having low reliability. The output function 34c stores the resultant reliability and the answer in the memory 33 in association with each other.

As described above, the medical information processing apparatus 30 according to the second modification of some embodiments extracts the first named entity from the prompt and extracts the second named entity from the answer to calculate the number of mutually corresponding named entities in the first named entity and the second named entity and determine reliability of the answer based on the number of the mutually corresponding named entities. In this manner, the medical information processing apparatus 30 according to the second modification of some embodiments can determine the answer reliability depending on the difference between the first attribute number and the second attribute number, using the first attribute number in the first named entity and the second attribute number in the second named entity. The medical information processing apparatus 30 according to the second modification of some embodiments can eliminate the necessity to perform searching for the first named entity and the second named entity, which leads to facilitating the answer reliability determination. Other effects of this modification are similar to or the same as those of some embodiments, therefore, a description thereof is omitted.

### Third Modification

A third modification concerns generating a prompt for making an inquiry about answer reliability (hereinafter, an inquiry prompt) on the basis of a prompt based on a user input (hereinafter, a user-input prompt) and an answer, to input the inquiry prompt to the LLM 11 or another generative model differing from the LLM 11 (hereinafter, a different LLM) and output answer reliability in accordance with an output of the LLM 11 or the different LLM.

The different LLM is stored in the memory of the information processing server 10. Alternatively, the different LLM may be stored in the memory of a cloud server different from the information processing server 10 or stored in the medical information processing apparatus 30. The different LLM corresponds to a generative AI different from the LLM 11. The different LLM is an AI model pre-trained using a large-scale corpus in the field of natural language processing. For example, upon receiving an input of a sentence (prompt) representing a question or an instruction, the different LLM is trained to generate and output a reply in line with the meaning of the input sentence.

The processor included in the information processing server 10 generates a reply by retrieving the different LLM from the memory and inputting, into the different LLM, the inquiry prompt output from the medical information processing apparatus 30. The processor then transmits the reply (output from the different LLM) to the medical information processing apparatus 30. The generated reply, **i.e.,** the answer for the inquiry prompt from the different LLM, contains medical information about the user. The LLM 11 can be incorporated in the medical information processing apparatus 30.

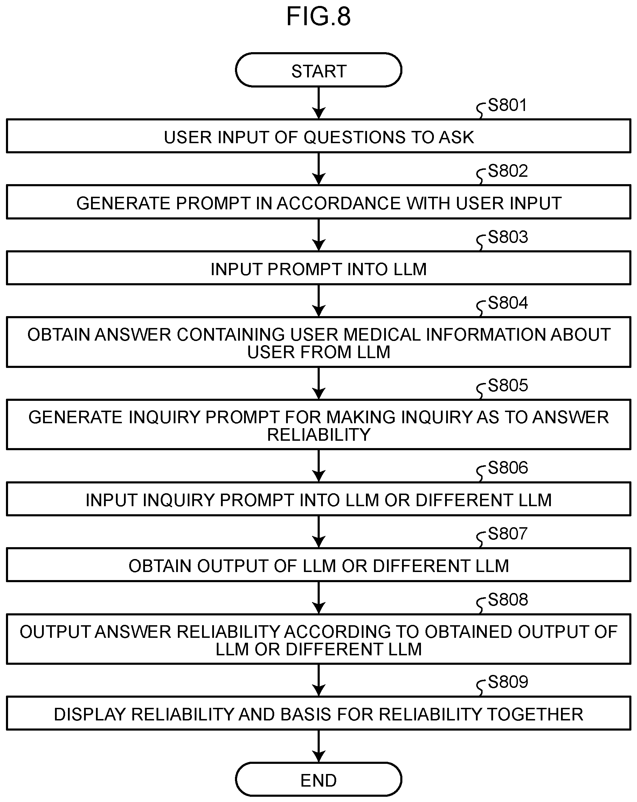

The following will describe a reliability determining process according to the third modification, referring to FIG. 8. FIG. 8 is a flowchart illustrating steps of a reliability determining process in the third modification, as an example. In the flowchart of FIG. 8, the processing in step S801 to step S804 and step S809 are the same as the processing in step S301 to step S304 and step S311 of FIG. 3, therefore, a description thereof is omitted.

### Reliability Determining Process

### Step S805

The processing circuitry 34 uses the generation function 34a to generate a prompt for making an inquiry (hereinafter, an inquiry prompt) as to answer reliability based on a user-input prompt and an answer for the user-input prompt.
Namely, the generation function 34a generates an inquiry prompt to be input to the LLM 11 or the different LLM. As an example, the user-input prompt may be such that **"I'm** a 30-year-old male and found a lump on my neck. Is this some kind of disease?", and the answer of the LLM 11 may be such that "A lump on the neck is usually benign so please observe the progress". In this case the inquiry prompt may include the user-input prompt, the answer, and an inquiry "How reliable is the LLM in this conversation?". The inquiry prompt generation can be implemented by any of known methods, therefore, a description thereof is omitted. The generation function 34a stores the inquiry prompt in the memory 33.

### Step S806

The processing circuitry 34 uses the obtaining function 34b to transmit the inquiry prompt to the information processing server 10. The information processing server 10 then inputs the inquiry prompt to the LLM 11 or the different LLM.

### Step S807

The information processing server 10 generates an answer for the input inquiry prompt (hereinafter, a reliability-related answer) as an output of the LLM 11 or the different LLM. The processing circuitry 34 uses the obtaining function 34b to obtain the output (reliability-related answer) of the LLM 11 or the different LLM from the information processing server 10. The obtaining function 34b stores the output and the inquiry prompt in the memory 33 in association with each other.

### Step S808

The processing circuitry 34 uses the output function 34c to determine reliability of the answer for the user-input prompt according to the obtained output (reliability-related answer) of the LLM 11 or the different LLM. When the reliability-related answer contains classification information for classifying reliability **(e.g.,** low, medium, high), the output function 34c outputs reliability of the answer for the user-input prompt, based on the classification information contained in the reliability-related answer.

As described above, the medical information processing apparatus 30 of the third modification of some embodiments generates an inquiry prompt for making an inquiry about reliability of the answer for the user input, based on the user-input prompt and the answer for the user-input prompt. The medical information processing apparatus 30 then obtains an output of the LLM 11 or the different LLM in response to an input of the inquiry prompt to the LLM 11 or the different LLM and determines the reliability of the answer in accordance with the obtained output of the LLM 11 or the different LLM.

As such, the medical information processing apparatus 30 of the third modification of some embodiments can determine the reliability of the answer based on the user-input prompt and the answer for the user-input prompt by using the LLM 11 or the different LLM. The medical information processing apparatus 30 of the third modification of some embodiments can eliminate the necessity to extract the first named entity and the second named entity, so that it can more simply determine the answer reliability. Other effects of this modification are similar to or the same as those of some embodiments, therefore, a description thereof is omitted.

### First Application

A first application represents any combination of some embodiments, the first modification, the second modification, and the third modification of some embodiments. According to the first application, the answer reliability, as determined by a combination of at least two of an embodiment, the first modification, the second modification, and the third modification of some embodiments, is displayed separately on the display 32 as a basis for the answer.

In addition, the processing circuitry 34 may use the output function 34c to determine general answer reliability by predetermined computation **(e.g.,** known weighted addition) of multiple degrees of reliability of the answer. In this case, the processing circuitry 34 may use the display control function 34d to display the general answer reliability on the display 32 along with the user-input prompt and the answer for the user-input prompt. The effects of the first application are the same as or similar to those of some embodiments, therefore, a description thereof is omitted.

To implement the technical idea of the present embodiment using the medical information processing system **1,** the medical information processing system 1 includes the generation function 34a that generates a prompt to be input to a generative model 11 in accordance with a user input; the obtaining function 34b that obtains an answer including medical information about the user from the generative model 11; and the output function 34c that outputs reliability of the answer based on the prompt and the answer. The steps of the reliability determining process performed by the medical information processing system 1 are in conformity with those in some embodiments. The effects of the medical information processing system 1 are the same as or similar to those of some embodiments. Thus, a description of the procedure and effects of the reliability determining process by the medical information processing system 1 is omitted.

To implement the technical idea of the present embodiment using an information processing apparatus, the information processing apparatus has the same configuration and structure as the information processing apparatus 30 of FIG. 1. The information processing apparatus includes the generation function 34a that generates a prompt to be input to the generative model 11 in accordance with a user input; the obtaining function 34b that obtains information about the user from the generative model 11 in response to an input of the prompt to the generative model 11; and the output function 34c that outputs reliability of the answer based on the prompt and the answer. The steps of the reliability determining process performed by the information processing apparatus are in conformity with those in some embodiments. The effects of the information processing apparatus are the same as or similar to those of some embodiments. Thus, a description of the procedure and effects of the reliability determining process by the information processing apparatus is omitted.

To implement the technical idea of the present embodiment by a medical information processing method, the medical information processing method includes generating a prompt to be input to the LLM 11 in accordance with a user input; inputting the prompt to the LLM 11; obtaining an answer including medical information about the user from the LLM 11; and outputting reliability of the answer based on the prompt and the answer. The steps of the reliability determining process implemented by the medical information processing method are in conformity with those in some embodiments. The effects of the medical information processing method are the same as or similar to those of some embodiments. Thus, a description of the procedure and effects of the reliability determining process by the medical information method is omitted.

To implement the technical idea of the present embodiment by an information processing method, the information processing method includes generating a prompt to be input to the LLM 11 in accordance with a user input; inputting the prompt to the LLM 11, obtaining an answer including medical information about the user from the LLM 11; and outputting reliability of the answer based on the prompt and the answer.

To implement the technical idea of the present embodiment by a medical information processing program, the medical information processing program causes a computer to perform generating a prompt to be input to the LLM 11 in accordance with a user input; inputting the prompt to the LLM 11, obtaining an answer including medical information about the user from the LLM 11; and outputting reliability of the answer based on the prompt and the answer. As an example, the reliability determining process can be implemented by installing the medical information processing program on a computer such as the information processing apparatus 30 of FIG. 1 and loading the program onto the memory. Such programs for causing the computer to perform the process can be stored for distribution in a storage medium such as a magnetic disk (e.g., a hard disk), an optical disk (e.g., a CD-ROM, a DVD), and a semiconductor memory.

Alternatively, the medical information processing program can be distributed using an electric communication function, for example, by downloading via the Internet, in addition to using the storage medium. The procedure of the medical information processing program are in conformity with the reliability determining process. The effects achieved by the medical information processing program are the same as or similar to those of some embodiments. Thus, a description of the procedure and effects of the reliability determining process by the medical information processing program is omitted.

### Second Application

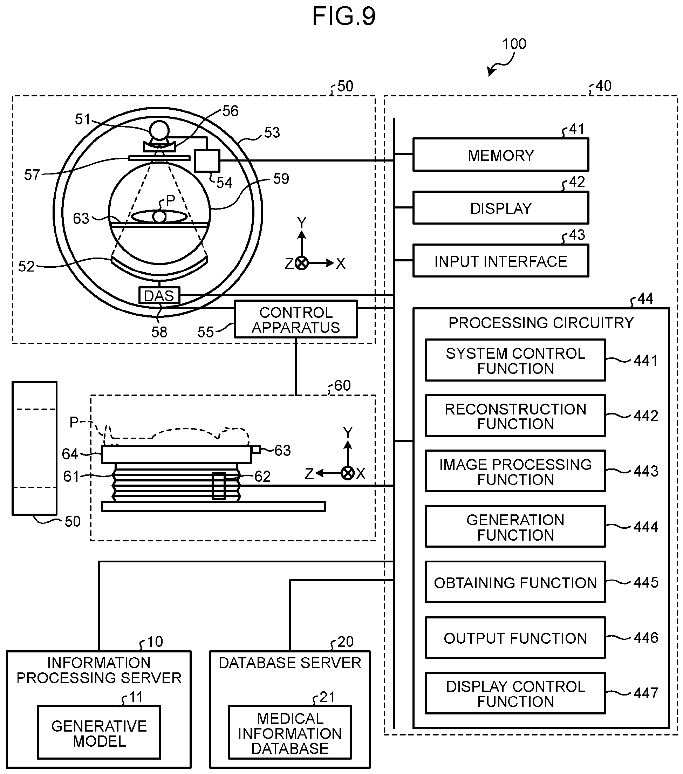

A second application concerns implementing the reliability determining process of some embodiments using an X-ray CT apparatus. FIG. 9 is a schematic block diagram illustrating an exemplary configuration of an X-ray computed tomography (CT) apparatus 100 according to the second application of some embodiments. The X-ray CT apparatus 1 is configured to irradiate a subject P with an X-ray from an X-ray tube 51 to detect the irradiation of the X-ray with an X-ray detector 52. The X-ray CT apparatus 100 generates CT images of the subject P based on an output from the X-ray detector 12.

As illustrated in FIG. 9, the X-ray CT apparatus 100 includes a gantry 50, a couch 60, and a console 40. FIG. 1 illustrates multiple gantries 50 for illustrative purposes. The gantry 50 is a scanning apparatus including elements for CT scans of the subject P with X-rays. The couch 60 is a carrier apparatus on which the subject P to be CT scanned is laid for positioning. The console 40 is a computer that performs control over the gantry 50. For example, the gantry 50 and the couch 60 are installed in a CT examination room while the console 40 is installed in a control room adjacent to the CT examination room. The gantry 50, the couch 60, and the console 40 are communicably connected to one another in a wired or wireless manner.

The console 40 may not be installed in the control room. For example, the console 40 may be installed together with the gantry 50 and the couch 60 in the same room. Alternatively, the console 40 may be incorporated in the gantry 50.

In the present embodiment, the rotational axis of a rotational frame 53 in a non-tilted state or in a longitudinal direction of a couch top 63 of the couch 60 is defined as a Z-axis direction. An axial direction orthogonal to the Z-axis direction and horizontal to the floor is defined as an X-axis direction. An axial direction orthogonal to the Z-axis direction and vertical to the floor is defined as a Y-axis direction.

As illustrated in FIG. 9, the gantry 50 includes the X-ray tube 51, the X-ray detector 52, the rotational frame 53, an X-ray high voltage apparatus 54, a control apparatus 55, a wedge 56, a collimator 57, and a data acquisition system (DAS) 58.

The X-ray tube 51 is a vacuum tube having a negative pole (filament) that generates thermoelectrons and a positive pole (target) that generates X-rays by colliding with the thermoelectrons. The X-ray tube 51 is supplied with a high voltage from the X-ray high-voltage apparatus 54 to generate X-rays. The X-ray tube 51 irradiates the subject P with X-rays by, for example, emitting the thermoelectrons from the negative pole to the positive pole.

The X-ray generating hardware is not limited to the X-ray tube 51. For example, in place of the X-ray tube 51, a fifth generation system may be used to generate X-rays. The fifth generation system may include a focus coil that focuses electronic beams generated from an electronic gun, a deflector coil for electromagnetic deflection of the electronic beams, and a target ring surrounding halfway around the subject P and with which the deflected electronic beams collide to thereby generate X-rays.

The X-ray detector 12 detects an X-ray emitted from the X-ray tube 51 and having passed through the subject P and outputs an electric signal corresponding to a dose of the detected X-ray to the DAS 58. Thus, the X-ray detector 52 detects an X-ray emitted from the X-ray tube 51 and having passed through the subject P. The X-ray detector 52 includes, for example, multiple arrays of detection elements arranged along a single arc about the focal point of the X-ray tube 51 in a channel direction. The X-ray detector 52 has a structure that arrays of detection elements in the channel direction are arrayed in a slice direction (column or row direction), for example. The X-ray detector 52 is exemplified by an indirect-conversion detector including a grid, a scintillator array, and an optical sensor array. The scintillator array includes multiple scintillators and each scintillator includes a scintillator crystal that outputs an amount of light corresponding to an amount of incident X-rays. The grid is disposed on the X-ray incident side of the scintillator array and includes an X-ray shield plate that functions to absorb scattered X-rays. The grid may be referred to as a collimator (one-dimensional collimator or two-dimensional collimator). The optical sensor array functions to convert an amount of light from the scintillators into an electric signal. The optical sensors may be, for example, photo multipliers (PMT). The X-ray detector 52 may be a direct-conversion detector including a semiconductor element that converts an incident X-ray into an electric signal. The X-ray detector 52 is an example of a detector unit.

The rotational frame 53 is an annular frame that supports the X-ray tube 51 and the X-ray detector 52 in opposing positions to rotate the X-ray tube 51 and the X-ray detector 52 under the control of the control apparatus 55 as later described. The rotational frame 53 is provided with an opening 59 with an image field of view (FOV) set. The rotational frame 53 is, for example, a casting made of aluminum. The rotational frame 53 can support the X-ray high voltage apparatus 54, the wedge 56, the collimator 57, and the DAS 58 in addition to the X-ray tube 51 and the X-ray detector 52. The rotational frame 53 can further support other various elements (not illustrated in FIG. 9).

The X-ray high voltage apparatus 54 includes a high voltage generator and an X-ray control device. The high voltage generator includes electric circuitry such as a transformer and a rectifier and functions to generate a high voltage to be applied to the X-ray tube 51 and a filament current to be supplied to the X-ray tube 51. The X-ray control device controls the output voltage in accordance with the X-rays emitted from the X-ray tube 51. The high-voltage generator may be a transformer type or an inverter type. Further, the X-ray high-voltage apparatus 54 may be disposed in the rotational frame 53 or the stationary frame (not illustrated) of the gantry 50. The stationary frame refers to a frame that rotatably supports the rotational frame 53.

The control apparatus 55 includes a driving mechanism such as a motor and an actuator, and processing circuitry including memory and a processor to control the driving mechanism. In accordance with an input signal from an input interface 43 or an input interface included in the gantry 50, the control apparatus 55 controls the operation of the gantry 50 and the couch 60. Examples of the operation control by the control apparatus 55 include the rotation control over the rotational frame 53, the tilting control over the gantry 50, and the operation control of the couch 60. The control apparatus 55 controls the gantry 50 to tilt by rotating the rotational frame 53 about an axis parallel to the X-axis direction according to tilt-angle information input through an input interface attached to the gantry 50. The control apparatus 55 may be included in the gantry 50 or in the console 40.

The wedge 56 is a filter for adjusting the amount of X-rays emitted from the X-ray tube 51. Specifically, the wedge 16 serves as a filter that allows the X-rays emitted from the X-ray tube 51 to transmit therethrough for attenuation, so that the subject P is irradiated with the X-rays from the X-ray tube 51 in a predefined distribution. The wedge 16 is formed of aluminum and has a predetermined target angle and a predetermined thickness. Examples of the wedge 16 include a wedge filter and a bow-tie filter.

The collimator 57 functions to limit the irradiation range of the X-rays having transmitted the wedge 56. The collimator 57 slidably supports lead plates forming slits and adjusts the form of the slits to shield the X-rays. The collimator 57 may be referred to as an X-ray diaphragm.

The DAS 58 functions to retrieve electric signals corresponding to the doses of X-rays detected by the X-ray detector 12 from the X-ray detector 52. The DAS 58 amplifies the electric signals and integrate or add them in a view period to acquire detection data having a digital value corresponding to the dose of X-rays during the view period. The detection data may be referred to as projection data. The DAS 58 can be implemented by, for example, an application specific integrated circuit (ASIC) incorporating a circuit element that can generate projection data. The projection data is transferred to the console 40 via a non-contact data transfer device, for example. The DAS 58 is an exemplary data acquirer unit.

The detection data generated by the DAS 58 is transmitted by optical communication from a transmitter with light emitting diodes (LED) disposed in the rotational frame 53 to a receiver with photodiodes disposed in the non-rotational part (e.g., stationary frame, not illustrated in FIG. 9) of the gantry 50, and then transferred to the console 40. Examples of adoptable detection-data transmission method from the rotational frame 53 to the non-rotational part of the gantry 50 include any non-contact data transfer method, in addition to the optical communication.

The present embodiment is described using the X-ray CT apparatus 100 incorporating the integrating X-ray detector 52 as an example. However, the techniques based on the present embodiment can be implemented as an X-ray CT apparatus 100 incorporating a photo-counting X-ray detector.

The couch 60 is a device on which the subject P to be scanned is to be laid and moved, and includes a base 61, a couch driver 62, the couch top 63, and a support frame 64. The base 61 is a housing that supports the support frame 34 movably in a vertical direction. The couch driver 62 is a drive mechanism that moves the couch top 63 on which the subject P is lying in the longitudinal direction of the couch top 63. The couch driver 62 includes a motor and an actuator. The couch top 63 is a plate on which the subject P is to be laid. The couch top 63 is placed on the top surface of the support frame 64. The couch top 63 can protrude from the couch 60 toward the gantry 50 so as to allow the entire body of the subject P to be scanned. The couch top 63 is formed of, for example, carbon fiber reinforced plastic (CFRP) with good X-ray transmissivity and good physical property such as rigidity and strength. For example, the couch top 63 is hollow inside. The support frame 64 supports the couch top 63 movably in the longitudinal direction of the couch top 63. The couch driver 62 may move the support frame 64 in the longitudinal direction of the couch top 63, in addition to the couch top 63.

The console 40 includes a memory 41, a display 42, the input interface 43, and processing circuitry 44. The processing circuitry 44, the memory 41, the display 42, and the input interface 43 perform data communications with one another via, for example, a bus. Although the console 40 and the gantry 50 are separately provided herein, the gantry 50 may include the console 40 or part of the elements of the console 40.

The memory 41 can be implemented by, for example, a semiconductor memory device as a ROM, a RAM, or a flash memory, a hard disk, or an optical disk. The memory 41 stores therein, for example, projection data and reconstructed image data (also referred to as CT image data). The storage regions of the memory 41 may be included in the X-ray CT apparatus 100 or in an external storage device connected via a network. The memory 41 is an exemplary storage unit.

The display 42 displays various kinds of information. For example, the display 42 displays medical images (CT images) generated by the processing circuitry 44 and a graphical user interface (GUI) that allows the operator to perform various kinds of operation. The information displayed on the display 42 includes images based on various kinds of medical image data such as mask images related to some embodiments and CT images on which mask images are superimposed. The display 42 can be any of various kinds of display when appropriate. Examples of the display 42 include a liquid crystal display (LCD), a cathode ray tube (CRT) display, an organic electroluminescence display (OELD), and a plasma display.

The display 42 may be installed in any location in the control room. The display 42 may be included in the gantry 50. The display 42 may be a desktop type or may include a tablet terminal wirelessly communicable with a body of the console 40. The display 42 can be one or two or more projectors. The display 42 is an exemplary display unit.

The input interface 43 receives various inputs from the operator to convert the inputs into electrical signals and output the electrical signals to the processing circuitry 44. As an example, the input interface 43 receives, from the operator, an acquisition condition for acquiring projection data, a reconstruction condition for reconstructing CT images, an image processing condition for generating post-processed images from CT images, and else.

Examples of the input interface 43 include a mouse, a keyboard, a trackball, a switch, a button, a joystick, a touch-pad, and a touch panel display, as appropriate. In the present embodiment the input interface 43 is not limited to the one including these physical operational components. Other examples of the input interface 43 include electrical-signal processing circuitry that receives an electrical signal corresponding to an input from an external input device separated from the apparatus to output the electrical signal to the processing circuitry 44. Alternatively, the input interface 43 may be included in the gantry 50. The input interface 43 may include a tablet terminal wirelessly communicable with the body of the console 40. The input interface 43 is an exemplary input unit.

The processing circuitry 44 controls the X-ray CT apparatus 100 as a whole. The processing circuitry 44 includes, for example, hardware resources including a processor and memory such as a ROM or a RAM. The processing circuitry 44 implements a system control function 441, a reconstruction function 442, an image processing function 443, a generation function 444, an obtaining function 445, an output function 446, and a display control function 447 by using the processor to load and execute the respective programs on the memory. The processing circuitry 44 is an exemplary processing unit.

The processing circuitry 44 uses the system control function 441 to control the respective functions of the processing circuitry 44 in response to receipt of inputs from the operator via the input interface 43. For example, the processing circuitry 44 controls CT scans in the gantry 50. The processing circuitry 44 obtains projection data resulting from CT scanning. Alternatively, the processing circuitry 44 may obtain detection data of the subject P from an outside of the X-ray CT apparatus 100.

The processing circuitry 44 uses the reconstruction function 442 to subject the detection data output from the DAS 58 to pre-processing including logarithm conversion, offset correction, sensitivity correction among the channels, and beam hardening correction, to generate data. The processing circuitry 44 stores the resultant data in the memory 41. Data before pre-processed **(i.e.,** detection data) and pre-processed data may be collectively referred to as projection data. The processing circuitry 44 perform reconstruction processing to the resultant projection data (pre-processed data) by filtered back projection (FBP), iterative reconstruction, or machine learning to generate CT image data.

Thus, the reconstruction function 442 reconstructs CT image data based on an output from the X-ray detector 52. The processing circuitry 44 stores the CT image data in the memory 41. The processing circuitry 44 implementing the reconstruction function 442 corresponds to a reconstruction unit.

The processing circuitry 44 uses the image processing function 443 to convert the CT image data generated by the reconstruction function 442 into planar image data or three-dimensional image data of any slice by a known method, in accordance with an operator's input received via the input interface 43. For example, the processing circuitry 44 subjects the CT image data to three-dimensional image processing including volume rendering, surface rendering, intensity projection, multi-planar reconstruction (MPR), and/or curved MPR (CPR) to generate rendering image data in some viewing direction. The reconstruction function 442 may directly generate three-dimensional image data such as the rendering image data in some viewing direction, **i.e.,** volume data. The processing circuitry 44 stores the planar image data and the three-dimensional image data in the memory 41.

Further, the processing circuitry 44 retrieves and executes the program corresponding to the generation function 444 from the memory 41. Thereby, the generation function 444 generates a prompt to be input to the LLM 11, in accordance with a user (for example, an attending physician of the subject P) input given via the input interface 43. For example, the generation function 444 generates a prompt including observation information as to the CT image data and to be input to a generative model (LLM 11). The generative model (LLM 11) is similar or identical to that of some embodiments, therefore, a description thereof is omitted.

Specifically, the generation function 444 obtains observation information, which is written in a report prepared based on the CT image data, from a picture archiving and communication system (PACS) server or a hospital information system (HIS) server (not illustrated). The generation function 444 generates a prompt including the observation information, in response to a user input given via the input interface 43. The generation function 444 performs the same functions as the generation function 34a of some embodiments shown in FIG. 1, therefore, a description thereof is omitted.

The processing circuitry 44 uses the obtaining function 445 to obtain an answer including medical information about the subject P from the generative model 11, in accordance with the prompt input to the generative model 11. Specifically, the processing circuitry 44 retrieves and executes the program corresponding to the obtaining function 445 from the memory 33. Thereby, the obtaining function 445 transmits the generated prompt to the information processing server 10 and the LLM 11 generates an answer in response to an input of the prompt into the LLM 11. Then, the obtaining function 445 obtains the answer from the LLM 11.

Specifically, the processor in the information processing server 10 inputs the prompt into the LLM 11 and generates an answer as an output of the LLM 11. The obtaining function 445 obtains the answer output from the LLM 11 from the information processing server 10. The answer output from the LLM 11 is responsive to the prompt input to the LLM 11. The obtaining function 445 performs the same functions as the obtaining function 34b of some embodiments shown in FIG. 1, therefore, a description thereof is omitted.

The processing circuitry 44 retrieves and executes the program corresponding to the output function 446 from the memory 33. Thereby, the output function 446 determines reliability of the answer based on the prompt and the answer. Specifically, the output function 446 extracts a named entity from the prompt by natural language processing. For example, when the prompt represents a sentence that "Observation information on CT image data output from the CT apparatus indicates a lump on the neck. Is this some kind of disease?", the named entity is represented by, for example, "site: neck, observation information: lump".

Further, the output function 446 extracts a named entity from the LLM 11's answer by natural language processing. For example, when the answer represents a sentence that "A lump on the neck is usually benign so please observe the progress", the named entity is represented by, for example, "site: neck, lesion: lump".

The named-entity extraction process can be implemented by known natural language processing, therefore, a description thereof is omitted. In addition, extracted named entities may be classified into a variety of items as described above. The data as to the extracted named entities may be structuralized as described above. Further, the processing circuitry 44 may perform the process of extracting a named entity (first named entity) from the prompt and extracting a named entity (second named entity) from the LLM 11's answer as a separate extraction process. The output function 446 performs the same functions as the output function 34c of some embodiments shown in FIG. 1, therefore, a description thereof is omitted. Thus, the reliability of the answer from the LLM 11 can be determined in the same manner as in some embodiments so that a description thereof is omitted.

The processing circuitry 44 uses the display control function 447 to display images on the display 42 based on various kinds of image data generated by the image processing function 443. The images displayed on the display 42 include CT images based on CT image data, planar images based on planar image data of any slice, and rendering images in some viewing direction based on rendering image data in some viewing direction, for example. The images displayed on the display 42 further include images depicting operation screens and images showing notices and warnings for the operator. The processing circuitry 44 implementing the display control function 447 is an exemplary display control unit.

In addition, the processing circuitry 44 uses the display control function 447 to display the answer of the LLM 11, the reliability of the answer, and the basis for the reliability together on the display 42. The display control function 447 performs the same functions as the display control function 34d of some embodiments in FIG. 1, therefore, a description thereof is omitted.

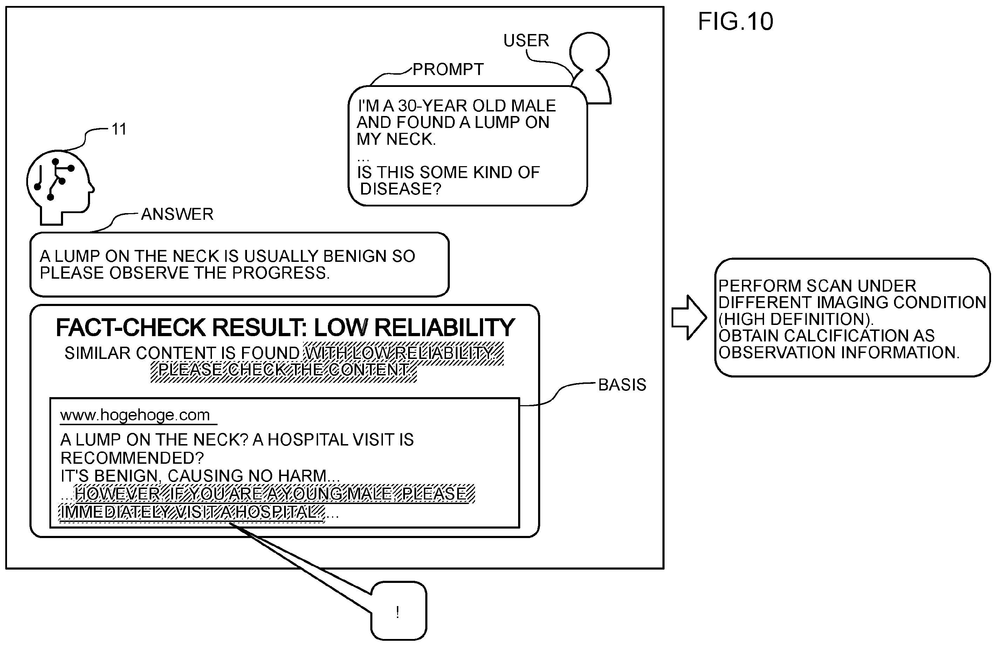

FIG. 10 illustrates a prompt, an LLM 11's answer for the prompt, reliability of the answer, a basis for the reliability by way of example. As illustrated in FIG. 10, the LLM 11's answer for the prompt contains medical information (e.g., benign, observe the progress in FIG. 10) of the subject P. As illustrated in FIG. 10, a site name in the medical information database 21 and wording representing a basis are displayed as the basis for the reliability, for example. At low reliability of the answer, a predetermined warning (e.g., exclamation mark) is added to the basis on the display, as illustrated in FIG. 10. This allows the user as an attending physician to easily understand the basis for the reliability in the event of low reliability of the answer.

Further, as illustrated in FIG. 10, the attending physician may change the imaging condition (scanning condition) for the subject P to, for example, a high definition in accordance with the reliability and the basis for the reliability. In this case the X-ray CT apparatus 100 scans the subject P again according to the high-definition imaging condition. As such, if the subject P is suspected to have calcification in the lesion, the X-ray CT apparatus 100 can obtain calcification as observation information. In addition, the reliability determining process in the second application can be appropriately iterated upon each scan and each obtainment of a report.

The respective functions 441 to 447 may not be implemented by a single piece of processing circuitry. The processing circuitry 44 may be constituted of a combination of multiple independent processors so that the individual processors execute the programs to implement the respective functions 441 to 447. Alternatively, the respective functions 441 to 447 may be implemented by one or two or more processing circuits in a distributed or integrated manner, when appropriate.

The console 40 has been described above as a single console that implements a plurality of functions, by way of example. However, multiple consoles may independently implement a plurality of functions. For example, multiple consoles may include the functions of the processing circuitry 44 such as the reconstruction function 442, the image processing function 443, the generation function 444, the obtaining function 445, the output function 446, and the display control function 447 in a distributed manner.

The processing circuitry 44 may be partially or entirely included in an integrated server that collectively processes detection data acquired by multiple medical image diagnosis apparatuses, in addition to being included in the console 40.

In addition, at least one of the post-processing, generation process, learning process, identifying process, or display process may be executed by either the console 40 or an external workstation or by both of the console 40 and the workstation concurrently. Examples of the workstation may include a computer including hardware resources such as a processor that implements the respective functions corresponding to the processes, and memory as a ROM or a RAM.

The X-ray CT image data may be reconstructed by either of a full-scan reconstruction and a half-scan reconstruction. For example, the reconstruction function 442 of the processing circuitry 44 uses 360-degree projection data of the subject P, **i.e.,** data on the entire circumference of the subject P, in the full-scan reconstruction. The processing circuitry 44 uses projection data of 180 degrees plus a fan angle in the half-scan reconstruction. In the following the processing circuitry 44 is defined to adopt the full-scan reconstruction that reconstructs CT image data from 360-degree projection data of the entire circumference of the subject P for the sake of simplicity.

The techniques based on the present embodiment are applicable to both a single-source X-ray computed tomography apparatus and a multiple-source X-ray computed tomography apparatus incorporating two or more pairs of X-ray tubes and detectors on the rotational ring.

Further, the techniques based on the present embodiment are applicable to an X-ray CT apparatus 100 adapted for dual-energy imaging. In this case, the X-ray high-voltage apparatus 54 is able to alternatively switch X-ray energy spectra emitted from the X-ray tube 51 by switching two voltage values at high speed, for example. In other words, the X-ray CT apparatus 100 is configured to be able to acquire projection data in each acquisition view while modulating the tube voltage at the timing conforming to a tube-voltage modulation control signal. By scanning the subject P at different tube voltages, the X-ray CT apparatus 100 can enhance the contrast density of CT images in accordance with the energy transmissivity of substance per **X-**ray energy spectrum.

Also, the X-ray CT apparatus 100 according to the present embodiment is defined to sequentially retrieve electric signals from the X-ray detector **52.**

The X-ray CT apparatus 100 according to the present embodiment can be structured as an upright **CT.** In such a case the X-ray CT apparatus 100 may include a patient support mechanism that is movable along the rotational axis of the rotational part of the gantry 50 while supporting the subject P in an upright state, in place of the movable couch top **53.** Alternatively, the X-ray CT apparatus 100 according to the present embodiment can be structured as a mobile CT in which the gantry 50 and the couch 60 are movable.

The reliability determining process of the second application and the effects thereof conform to those of some embodiments, therefore, a description thereof is omitted.

According to at least one of the embodiments, modifications, and applications as above, it is made possible to improve the reliability of answers of the generative model 11 applied to the field of medicine in terms of accuracy.

While certain embodiments have been described, these embodiments have been presented by way of example only, and are not intended to limit the scope of the inventions. Indeed, the novel embodiments described herein may be embodied in a variety of other forms; furthermore, various omissions, substitutions and changes in the form of the embodiments described herein may be made without departing from the scope of the invention as defined by the appended claims.

## Claims

1. A medical information processing apparatus (30) comprising:
a generator unit (34a) configured to generate a prompt to be input to a first generative model (11), in accordance with a user's input,
an obtaining unit (34b) configured to obtain an answer including medical information from the first generative model (11), in response to an input of the prompt to the first generative model (11), and
an output unit (34c) configured to output reliability of the answer based on the prompt and the answer.

2. The medical information processing apparatus (30) according to claim **1,** wherein the output unit (34c) is configured to:
extract a first named entity from the prompt,
extract a second named entity from the answer,
search a medical information database (21) using the first named entity and the second named entity as a search condition, to identify document data based on how many times the first named entity and the second named entity appear,
calculate a similarity of the document data relative to the prompt and the answer, based on a first vector and a second vector, the first vector representing a vectorization of the document data, the second vector representing a vectorization of the prompt and the answer, and
determine the reliability based on the similarity.

3. The medical information processing apparatus (30) according to claim **2,** wherein
the output unit (34c) is configured to identify document data in which the first named entity and the second named entity appear a largest number of times.

4. The medical information processing apparatus (30) according to claim **1,** wherein the output unit (34c) is configured to:
extract a first named entity from the prompt,
extract a second named entity from the answer,
search a medical information database (21) using the first named entity and the second named entity as a search condition, to calculate a hit count under the search condition, and
determine the reliability based on the hit count.

5. The medical information processing apparatus (30) according to any one of claims 1 to **4,** wherein the output unit (34c) is configured to:
extract a first named entity from the prompt,
extract a second named entity from the answer,
calculate a number of mutually corresponding named entities in the first named entity and the second named entity, and
determine the reliability based on the number of mutually corresponding named entities.

6. The medical information processing apparatus (30) according to claim 5, wherein
the generator unit (34a) is configured to generate an inquiry prompt for inquiring about reliability of the answer, based on the prompt and the answer,
the obtaining unit (34b) is configured to obtain an output from the first generative model (11) or a second generative model different from the first generative model (11), in response to an input of the inquiry prompt to the first generative model (11) or the second generative model, and
the output unit (34c) is configured to output the reliability in accordance with the obtained output from the first generative model (11) or the second generative model.

7. The medical information processing apparatus (30) according to any one of claims 1 to 4, wherein
the generator unit (34a) is configured to generate an inquiry prompt for inquiring about reliability of the answer,
the obtaining unit (34b) is configured to obtain an output from the first generative model (11) or a second generative model different from the first generative model (11), in response to an input of the inquiry prompt, the prompt, and the answer to the first generative model (11) or the second generative model, and
the output unit (34c) is configured to output the reliability in accordance with the obtained output from the first generative model (11) or the second generative model.

8. The medical information processing apparatus (30) according to any one of claims 1 to 4, further comprising:
a display unit (42) that displays the reliability and a basis for the reliability.

9. The medical information processing apparatus (30) according to any one of claims 1 to 4, further comprising:
a display unit (42) that displays the prompt and the answer with the reliability.

10. A medical information processing method comprising:
generating a prompt to be input to a generative model (11), in accordance with a user's input;
inputting the prompt to the generative model (11);
obtaining an answer including medical information from the generative model (11); and
outputting reliability of the answer based on the prompt and the answer.

11. A medical information processing program for causing a computer to perform:
generating a prompt to be input to a generative model (11), in accordance with a user's input;
inputting the prompt to the generative model (11);
obtaining an answer including medical information from the generative model (11); and
outputting reliability of the answer based on the prompt and the answer.
